# EUROPEAN PATENT APPLICATION

(11) **EP 3 173 012 A1**
(43) Date of publication of application: **31.05.2017**
(21) Application number: 16782842.5
(22) Date of filing: 05.02.2016
(51) Int. Cl.: A61B 1/04, A61B 1/06, G02B 23/24

(54) **LIVING BODY OBSERVATION SYSTEM**

(30) Priority: 21.04.2015 JP 2015086978
(71) Applicant: Olympus Corporation, Tokyo 192-8507 (JP)
(72) Inventor: IGARASHI, Makoto, Hachioji-shi Tokyo 192-8507 (JP); YAJIMA, Hiroyoshi, Hachioji-shi Tokyo 192-8507 (JP)
(74) Representative: Gunzelmann, Rainer
(86) International application number: PCT/JP2016/053496
(87) International publication number: WO 2016/170820

(57) **Abstract**

A biological observation system includes: a light source section configured to supply illumination light applied to a region to be observed including biological tissue, the illumination light including first illumination light that is one of red light and near infrared light in a visible region and second illumination light that is light in the visible region different from the first illumination light; a light detection section configured to receive return light emitted from the region to be observed and output an output signal; a signal processing section configured to output a frequency spectrum signal indicating a frequency spectrum obtained by analyzing a frequency of the return light emitted from the region to be observed according to application of the first illumination light; an image generation section configured to generate an intensity image based on the output signal and generate a frequency spectrum image based on the frequency spectrum signal; and a display control section configured to generate and display an observation image of the region to be observed by using the intensity image and the frequency spectrum image.

## Description

### Technical Field

The present invention relates to a biological observation system, and particularly, to a biological observation system usable in conducting a surgical treatment of biological tissue in a body cavity.

### Background Art

Various techniques usable in conducting a surgical treatment of biological tissue in a body cavity have been conventionally proposed.

More specifically, for example, International Publication No. WO 2013/134411 discloses a configuration including a treatment instrument used in conducting a surgical treatment of biological tissue in a body cavity, wherein an optical sensor arranged on a position close to a surgical field during surgery and configured to receive return light from the surgical field to which light from a light source is applied is provided on the treatment instrument, and characteristics of blood vessels existing inside of the biological tissue in the surgical field are acquired based on a sensing signal outputted from the optical sensor.

However, according to the configuration disclosed in International Publication No. WO 2013/134411, a detection range of the optical sensor provided on the treatment instrument is narrow, and for example, there is a problem that a distribution state of desirable blood vessels existing in a deep part of the biological tissue in the surgical field cannot be checked all at once. Therefore, according to the configuration disclosed in International Publication No. WO 2013/134411, there is an issue corresponding to the problem that an excessive burden is imposed on a surgeon conducting the surgical treatment of the biological tissue in the body cavity, for example.

The present invention has been made in view of the circumstances, and an object of the present invention is to provide a biological observation system that can reduce a burden of a surgeon conducting a surgical treatment of biological tissue in a body cavity.

### Disclosure of Invention

### Means for Solving the Problem

An aspect of the present invention provides a biological observation system including: a light source section configured to supply illumination light applied to a region to be observed including biological tissue, the illumination light including at least first illumination light that is light of one of red light and near infrared light in a visible region and second illumination light that is light in the visible region different from the first illumination light; a light detection section configured to receive return light emitted from the region to be observed according to application of the illumination light and output an output signal according to intensity of the received return light; a signal processing section configured to analyze a frequency of the output signal outputted from the light detection section to extract a frequency spectrum signal indicating a frequency spectrum of the return light emitted from the region to be observed according to application of the first illumination light; an image generation section configured to generate one or more intensity images based on the output signal outputted from the light detection section according to the application of the illumination light and generate a frequency spectrum image based on the frequency spectrum signal outputted from the signal processing section according to the application of the first illumination light; and a display control section configured to perform action for generating and displaying an observation image of the region to be observed by using at least one intensity image of the one or more intensity images and the frequency spectrum image.

### Brief Description of the Drawings

Fig. 1 is a diagram showing a configuration of main parts of a biological observation system according to an embodiment;
Fig. 2 is a diagram for describing an example of a configuration of a light source section of the biological observation system according to the embodiment;
Fig. 3 is a cross-sectional view for describing a configuration of an actuator section provided on an optical scanning probe of the biological observation system according to the embodiment;
Fig. 4 is a diagram for describing an example of a configuration of a light detection section of the biological observation system according to the embodiment;
Fig. 5 is a diagram for describing an example of a configuration of an image processing section of the biological observation system according to the embodiment;
Fig. 6 is a diagram schematically showing an example of an observation image displayed on a display apparatus of the biological observation system according to the embodiment;
Fig. 7 is a diagram schematically showing an example of an image generated by the image processing section of the biological observation system according to the embodiment;
Fig. 8 is a diagram schematically showing an example of an image generated by the image processing section of the biological observation system according to the embodiment;
Fig. 9 is a diagram schematically showing an example of an image generated by the image processing section of the biological observation system according to the embodiment;
Fig. 10 is a diagram schematically showing an example of an image generated by the image processing section of the biological observation system according to the embodiment;
Fig. 11 is a diagram schematically showing an example of an observation image displayed on the display apparatus of the biological observation system according to the embodiment;
Fig. 12 is a diagram for describing an example of a configuration of a light detection section of the biological observation system according to a modification of the embodiment; and
Fig. 13 is a diagram for describing an example of a configuration of an image processing section of the biological observation system according to the modification of the embodiment.

### Best Mode for Carrying Out the Invention

Hereinafter, an embodiment of the present invention will be described with reference to the drawings.

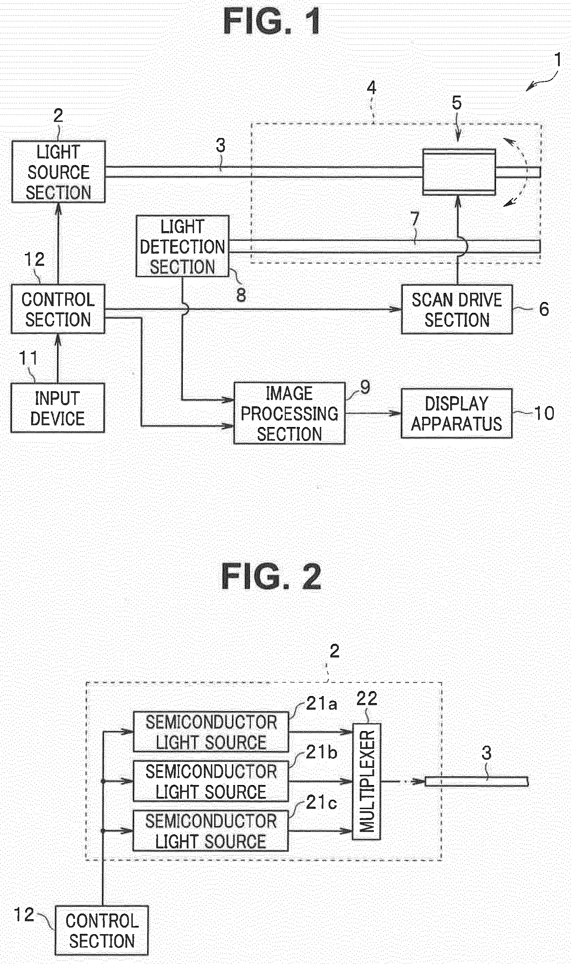

Figs. 1 to 13 relate to the embodiment of the present invention. Fig. 1 is a diagram showing a configuration of main parts of a biological observation system according to the embodiment.

A biological observation system 1 includes, for example, a light source section 2, an optical fiber 3, an optical scanning probe 4, an actuator section 5, a scan drive section 6, an optical fiber bundle 7, a light detection section 8, an image processing section 9, a display apparatus 10, an input device 11, and a control section 12 as shown in Fig. 1.

The light source section 2 can generate illumination light for illuminating a region to be observed including biological tissue and supply the illumination light to an incident end portion of the optical fiber 3. As shown in Fig. 2, the light source section 2 includes semiconductor light sources 21 a, 21 b, and 21c and a multiplexer 22. Fig. 2 is a diagram for describing an example of a configuration of the light source section of the biological observation system according to the embodiment.

The semiconductor light source 21 a includes, for example, and LD (laser diode) or an LED (light-emitting diode) and can be turned on or off according to control by the control section 12. When the semiconductor light source 21a is turned on according to the control by the control section 12, the semiconductor light source 21a is configured to emit B light to the multiplexer 22, wherein the B light is light in which a center wavelength belongs to a blue region.

The semiconductor light source 21b includes, for example, an LD or an LED and can be turned on or off according to control by the control section 12. When the semiconductor light source 21b is turned on according to the control by the control section 12, the semiconductor light source 21b is configured to emit G light to the multiplexer 22, wherein the G light is light in which a center wavelength belongs to a green region.

The semiconductor light source 21c includes, for example, an LD or an LED and can be turned on or off according to control by the control section 12. When the semiconductor light source 21c is turned on according to the control by the control section 12, the semiconductor light source 21c is configured to emit R light to the multiplexer 22, wherein the R light is light in which a center wavelength belongs to a red region to a near infrared region (for example, 600 nm to 1000 nm). Note that in the present embodiment, preferably, the center wavelength of the R light can be one of 630 nm, 670 nm, 780 nm, and 940 nm.

The multiplexer 22 can multiplex the B light emitted from the semiconductor light source 21 a, the G light emitted from the semiconductor light source 21b, and the R light emitted from the semiconductor light source 21c and emit the light to the incident end portion of the optical fiber 3.

That is, the light source section 2 can separately or simultaneously supply the R light that is light of one of red light and near infrared light in a visible region, the G light that is light in the visible region different from the R light, and the B light that is light in the visible region different from both of the R light and the G light, as the illumination light applied to the region to be observed including the biological tissue.

The optical fiber 3 is configured by, for example, a single mode fiber. The incident end portion of the optical fiber 3 including a light incident surface is connected to the light source section 2. An emission end portion of the optical fiber 3 including a light emission surface is arranged on a distal end portion of the optical scanning probe 4. That is, the optical fiber 3 can transmit the illumination light supplied from the light source section 2 and emit the transmitted illumination light to the region to be observed from the emission end portion.

The optical scanning probe 4 has an elongated shape that allows insertion into a body cavity of the subject. The optical fiber 3 and the optical fiber bundle 7 are respectively inserted into the optical scanning probe 4. The actuator section 5 configured to swing the emission end portion of optical fiber 3 according to a drive signal supplied from the scan drive section 6 is provided inside of the optical scanning probe 4.

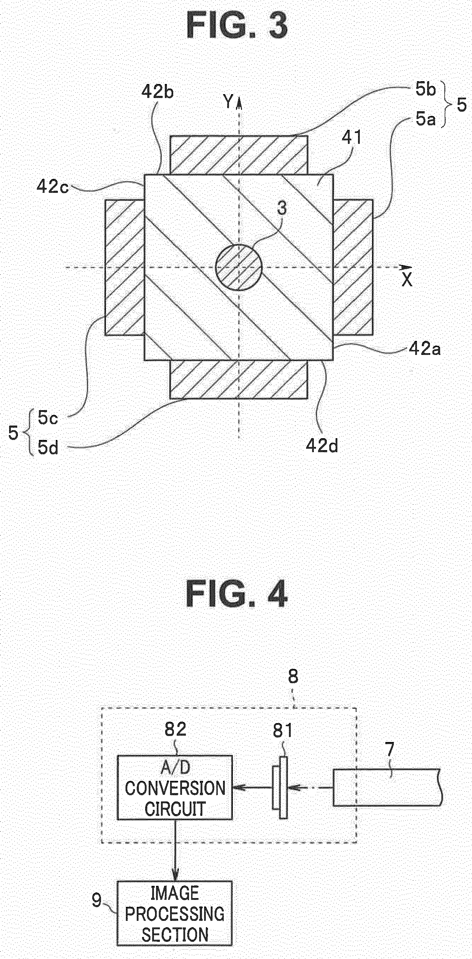

The optical fiber 3 and the actuator section 5 are respectively arranged to have, for example, a positional relationship shown in Fig. 3 in a cross section perpendicular to a longitudinal axis direction of the optical scanning probe 4. Fig. 3 is a cross-sectional view for describing a configuration of the actuator section provided on the optical scanning probe of the biological observation system according to the embodiment.

As shown in Fig. 3, a ferrule 41 as a bonding member is arranged between the optical fiber 3 and the actuator section 5. More specifically, the ferrule 41 is formed by, for example, zirconia (ceramic) or nickel.

As shown in Fig. 3, the ferrule 41 is formed as a quadrangular prism and includes: side surfaces 42a and 42c perpendicular to an X axis direction that is a first axis direction orthogonal to the longitudinal axis direction of the optical scanning probe 4; and side surfaces 42b and 42d perpendicular to a Y axis direction that is a second axis direction orthogonal to the longitudinal axis direction of the optical scanning probe 4. The optical fiber 3 is fixed and arranged at a center of the ferrule 41. Note that the ferrule 41 may be formed in a shape other than the quadrangular prism as long as the ferrule 41 has a pillar shape.

The actuator section 5 can swing the emission end portion of the optical fiber 3 based on the drive signal supplied from the scan drive section 6 to displace, along a predetermined scan path, an application position of the illumination light emitted to the region to be observed through the emission end portion. As shown in Fig. 3, the actuator section 5 includes a piezoelectric device 5a arranged along the side surface 42a, a piezoelectric device 5b arranged along the side surface 42b, a piezoelectric device 5c arranged along the side surface 42c, and a piezoelectric device 5d arranged along the side surface 42d.

The piezoelectric devices 5a to 5d have directions of polarization individually set in advance and are configured to expand and contract according to the drive signal supplied from the scan drive section 6.

The scan drive section 6 includes, for example, a drive circuit. The scan drive section 6 is configured to generate a drive signal for driving the actuator section 5 based on control by the control section 12 and supply the generated drive signal to the actuator section 5.

The optical fiber bundle 7 is configured by, for example, bundling a plurality of optical fibers. An incident end portion of the optical fiber bundle 7 is arranged on a distal end portion of the optical scanning probe 4. An emission end portion of the optical fiber bundle 7 is connected to the light detection section 8. That is, the optical fiber bundle 7 is configured to receive return light emitted from the region to be observed according to application of the illumination light emitted through the optical fiber 3 and is configured to be able to transmit the received return light to the light detection section 8.

The light detection section 8 is configured to receive the return light entered through the emission end portion of the optical fiber bundle 7, generate electrical signals according to intensity of the received return light, convert the generated electrical signals to digital signals, and sequentially output the digital signals. That is, the light detection section 8 is configured to receive the return light emitted from the region to be observed according to application of the illumination light to the region to be observed and output output signals according to the intensity of the received return light. The light detection section 8 includes, for example, a light detection device 81 and an A/D conversion circuit 82 as shown in Fig. 4. Fig. 4 is a diagram for describing an example of a configuration of the light detection section of the biological observation system according to the embodiment.

The light detection device 81 includes, for example, a PD (photodiode) or an APD (avalanche photodiode). The light detection device 81 is configured to receive the return light entered through the emission end portion of the optical fiber bundle 7, generate electrical signals according to the intensity of the received return light, and sequentially output the electrical signals to the A/D conversion circuit 82.

The A/D conversion circuit 82 is configured to convert the electrical signals outputted from the light detection device 81 to digital signals and sequentially output the digital signals to the image processing section 9.

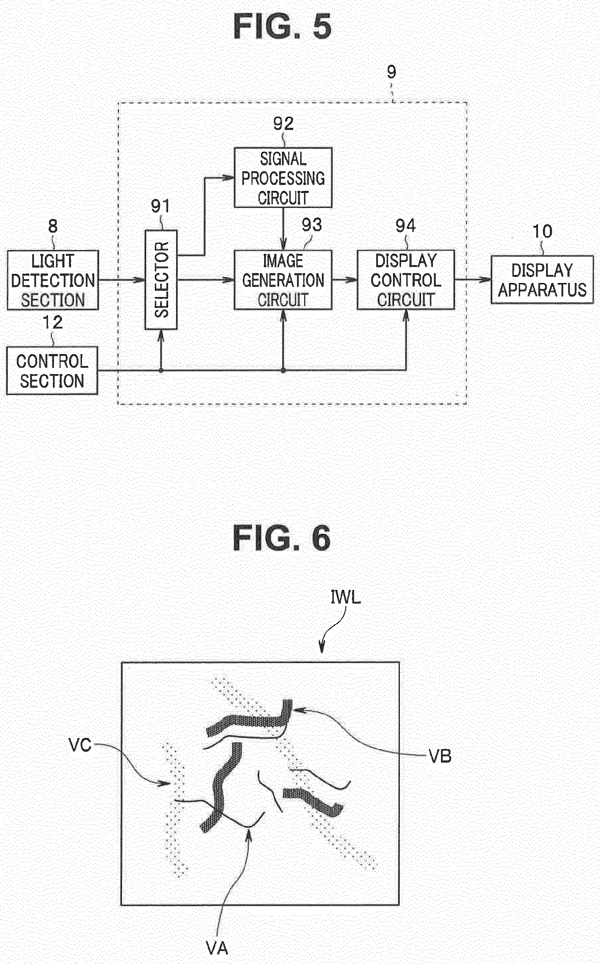

The image processing section 9 is configured to generate an observation image by executing a mapping process or the like of mapping the digital signals sequentially outputted from the light detection section 8 as pixel information based on control by the control section 12 and output the generated observation image to the display apparatus 10. The image processing section 9 includes, for example, a selector 91, a signal processing circuit 92, an image generation circuit 93, and a display control circuit 94 as shown in Fig. 5. Fig. 5 is a diagram for describing an example of a configuration of the image processing section of the biological observation system according to the embodiment.

The selector 91 can perform action for switching an output destination of the digital signals inputted from the light detection section 8 based on control by the control section 12.

The signal processing circuit 92 is configured to apply a process, such as DFT (discrete Fourier transform) and FFT (fast Fourier transform), to the digital signals outputted from the selector 91 to generate frequency spectrum signals indicating frequency spectra obtained by analyzing frequencies of the return light emitted from the region to be observed according to application of the illumination light and to output the generated frequency spectrum signals to the image generation circuit 93.

When the region to be observed is scanned in a predetermined scan path for example, the image generation circuit 93 is configured to specify pixel positions in a raster format corresponding to application positions of the illumination light in the predetermined scan path based on control by the control section 12. The image generation circuit 93 is configured to generate an image (hereinafter, also called an intensity image) of one field by executing a mapping process or the like of mapping, on the specified pixel positions, pixel information that is luminance values according to sizes of gradation values of the digital signals sequentially outputted from the selector 91 and output the generated image to the display control circuit 94.

When the region to be observed is scanned in a predetermined scan path for example, the image generation circuit 93 is configured to specify pixel positions in a raster format corresponding to application positions of the illumination light in the predetermined scan path based on control by the control section 12. The image generation circuit 93 is configured to generate an image (hereinafter, also called a frequency spectrum image) of one field by executing a mapping process or the like of mapping, on the specified pixel positions, pixel information that is luminance values according to average values or integrated values of sizes of frequency spectra indicated by the frequency spectrum signals sequentially outputted from the signal processing circuit 92 and output the generated image to the display control circuit 94.

The display control circuit 94 is configured to use respective images outputted from the image generation circuit 93 and allocate the images to a red channel (hereinafter, also called an R channel), a green channel (hereinafter, also called a G channel), and a blue channel (hereinafter, also called a B channel) of the display apparatus 10 to generate an observation image of one frame and to output the generated observation image to the display apparatus 10 based on control by the control section 12. That is, the display control circuit 94 can perform action for generating and displaying the observation image of the region to be observed by using at least one intensity image of one or more intensity images outputted from the image generation circuit 93 and the frequency spectrum image.

The display apparatus 10 includes, for example, a liquid crystal display. The display apparatus 10 can display the observation image and the like outputted from the image processing section 9.

The input device 11 includes, for example, a user interface, such as a switch and/or a button that can be operated by a user. The input device 11 can also issue various instructions according to operation by the user to the control section 12. More specifically, the input device 11 is provided with, for example, a display mode selector switch (not shown) that is a switch capable of issuing an instruction for setting a display mode of the observation image displayed on the display apparatus 10 to one display mode selected from a plurality of display modes.

The control section 12 includes a control circuit such as a CPU.

The control section 12 is configured to control the light source section 2 to apply the R light, the G light, and the B light to the region to be observed in a predetermined illumination pattern and control the image processing section 9 to switch the output destination of the digital signals inputted to the selector 91 according to the predetermined illumination pattern.

The control section 12 is configured to control the scan drive section 6 to generate drive signals used to two-dimensionally scan the region to be observed in a predetermined scan path and control the image processing section 9 to cause the image generation circuit 93 to execute the mapping process according to the predetermined scan path.

More specifically, the control section 12 can control the scan drive section 6 to generate, for example, a drive signal DSX with a signal waveform obtained by applying a predetermined modulation to a sine wave and a drive signal DSY with a signal waveform shifting a phase of the first drive signal by 90°, as drive signals used to scan the region to be observed in a spiral scan path. Note that when the control is performed, the region to be observed can be two-dimensionally scanned in the spiral scan path by supplying the drive signal DSX generated by the scan drive section 6 to the piezoelectric devices 5a and 5c and supplying the drive signal DSY generated by the scan drive section 6 to the piezoelectric devices 5b and 5d, for example.

That is, the optical scanning probe 4 is configured to scan the region to be observed by displacing the application positions of the R light, the G light, and the B light supplied from the light source section 2 along the predetermined scan path in a spiral shape or the like based on the drive signals supplied from the scan drive section 6 according to the control by the control section 12 and is configured to transmit the return light emitted from the region to be observed to the light detection section 8.

The control section 12 is configured to control the image processing section 9 to set a method by the display control circuit 94 allocating images to respective color channels of the display apparatus 10 so that the observation image corresponding to the display mode set in the display mode selector switch of the input device 11 is displayed. That is, the control section 12 is configured to control the image processing section 9 to display the observation image corresponding to the display mode set in the display mode selector switch of the input device 11.

Subsequently, an effect of the biological observation system 1 configured as described above will be described.

After connecting each section of the biological observation system 1 and applying power, the user, such as a surgeon turns on, for example, a scan start switch (not shown) of the input device 11 to instruct the control section 12 to start scanning of the region to be observed by the optical scanning probe 4. After connecting each section of the biological observation system 1 and applying power, the user operates the display mode selector switch of the input device 11 to instruct the control section 12 to set a display mode MW for displaying a white light image as an observation image.

When the control section 12 senses that the scan start switch is turned on and senses that the display mode MW is set, the control section 12 controls the light source section 2 to illuminate the region to be observed in an illumination pattern IPK in which the R light, the G light, and the B light are applied in time division, and the application frequency of the R light, the application frequency of the G light, and the application frequency of the B light are the same, for example. According to the control according to the illumination pattern IPK, the region to be observed is illuminated in a period of scanning for generating an observation image of one frame such that the number of times of application of the R light: the number of times of application of the G light: the number of times of application of the B light = 1 : 1 : 1.

The control section 12 controls the image processing section 9 to switch the output destinations of the digital signals inputted to the selector 91 according to the illumination pattern IPK.

More specifically, the control section 12 controls the image processing section 9 to set, in the image generation circuit 93, the output destinations of the digital signals inputted to the selector 91 according to the application timing of the R light, the G light, and the B light in the illumination pattern IPK, for example.

When the control section 12 senses that the scan start switch is turned on, the control section 12 controls the scan drive section 6 to generate drive signals used to two-dimensionally scan the region to be observed in a scan path SP and controls the image processing section 9 to cause the image generation circuit 93 to execute a mapping process according to the scan path SP.

According to the control by the control section 12 described above, the illumination light according to the illumination pattern IPK is applied along the scan path SP to two-dimensionally scan the region to be observed. Return light emitted from the region to be observed according to application of the illumination light enters the light detection section 8 through the optical fiber bundle 7, and digital signals generated according to the intensity of the return light are sequentially outputted to the image processing section 9.

According to the control by the control section 12 described above, digital signals DRS generated by the light detection section 8 at the application timing of the R light in the illumination pattern IPK are outputted to the image generation circuit 93 through the selector 91. Digital signals DGS generated by the light detection section 8 at the application timing of the G light in the illumination pattern IPK are outputted to the image generation circuit 93 through the selector 91. Digital signals DBS generated by the light detection section 8 at the application timing of the B light in the illumination pattern IPL are outputted to the image generation circuit 93 through the selector 91.

Based on control by the control section 12, the image generation circuit 93 specifies the pixel positions in the raster format corresponding to the application positions of the R light in the scan path SP, generates an intensity image PR of one field by executing a mapping process or the like of mapping, on the specified pixel positions, pixel information that is luminance values according to sizes of gradation values of the digital signals DRS sequentially outputted from the selector 91, and outputs the generated intensity image PR to the display control circuit 94.

Based on control by the control section 12, the image generation circuit 93 specifies the pixel positions in the raster format corresponding to the application positions of the G light in the scan path SP, generates an intensity image PG of one field by executing a mapping process or the like of mapping, on the specified pixel positions, pixel information that is luminance values according to sizes of gradation values of the digital signals DGS sequentially outputted from the selector 91, and outputs the generated intensity image PG to the display control circuit 94.

Based on control by the control section 12, the image generation circuit 93 specifies the pixel positions in the raster format corresponding to the application positions of the B light in the scan path SP, generates an intensity image PB of one field by executing a mapping process or the like of mapping, on the specified pixel positions, pixel information that is luminance values according to sizes of gradation values of the digital signals DBS sequentially outputted from the selector 91, and outputs the generated intensity image PB to the display control circuit 94.

When the control section 12 senses that the display mode MW is set, the control section 12 controls the image processing section 9 to allocate the intensity image PR to the R channel of the display apparatus 10, allocate the intensity image PG to the G channel of the display apparatus 10, and allocate the intensity image PB to the B channel of the display apparatus 10.

When the display mode MW is set, the display control circuit 94 generates an observation image of one frame by performing action of, for example, allocating the intensity image PR of one field outputted from the image generation circuit 93 to the R channel of the display apparatus 10, allocating the intensity image PG of one field outputted from the image generation circuit 93 to the G channel of the display apparatus 10, and allocating the intensity image PB of one field outputted from the image generation circuit 93 to the B channel of the display apparatus 10 and outputs the generated observation image to the display apparatus 10 based on the control by the control section 12.

On the other hand, the user inserts the optical scanning probe 4 into a body cavity of the subject while checking a white light image displayed on the display apparatus 10 at the setting of the display mode MW to arrange the distal end portion of the optical scanning probe 4 on a position that allows scanning desirable biological tissue LM in the body cavity as a region to be observed. In response to the operation by the user, a white light image IWL is displayed as an observation image on the display apparatus 10, the white light image IWL including a blood vessel VA with a small diameter existing in the mucosal layer of the desirable biological tissue LM, a blood vessel VB existing in the submucosa of the desirable biological tissue LM, and a blood vessel VC with a large diameter existing in the muscularis propria of the desirable biological tissue LM as shown for example in Fig. 6. Fig. 6 is a diagram schematically showing an example of the observation image displayed on the display apparatus of the biological observation system according to the embodiment.

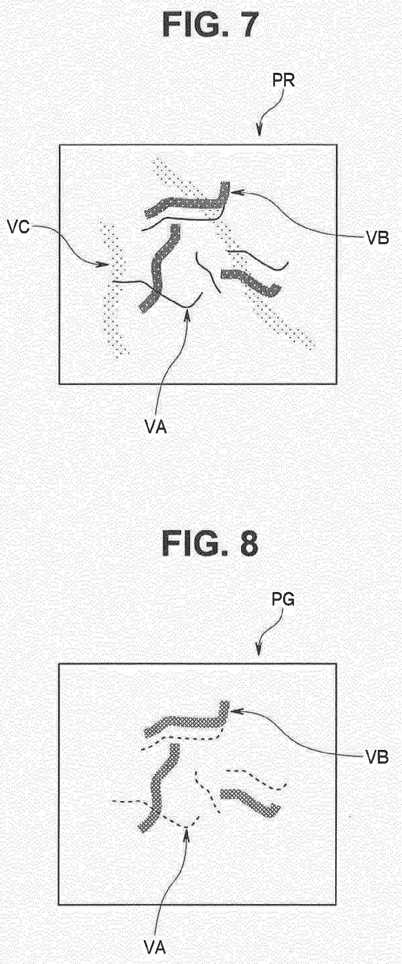

According to the action and the like of each section of the biological observation system 1 described above, when the desirable biological tissue LM is two-dimensionally scanned in the display mode MW, the image generation circuit 93 generates an intensity image PR with luminance values according to sizes of absorption and scattering of the R light by the blood flowing through the blood vessels VA, VB, and VC, an intensity image PG with luminance values according to sizes of absorption and scattering of the G light by the blood flowing through the blood vessels VA and VB, and an intensity image PB with luminance values according to sizes of absorption and scattering of the B light by the blood flowing through the blood vessel VA as shown for example in Figs. 7 to 9. Therefore, the white light image IWL displayed on the display apparatus 10 when the display mode MW is set is displayed as an image in which the blood vessels VA and VB can be visually recognized, but visually checking the blood vessel VC is difficult as illustrated in Fig. 6. Figs. 7, 8, and 9 are diagrams schematically showing examples of images generated by the image processing section of the biological observation system according to the embodiment.

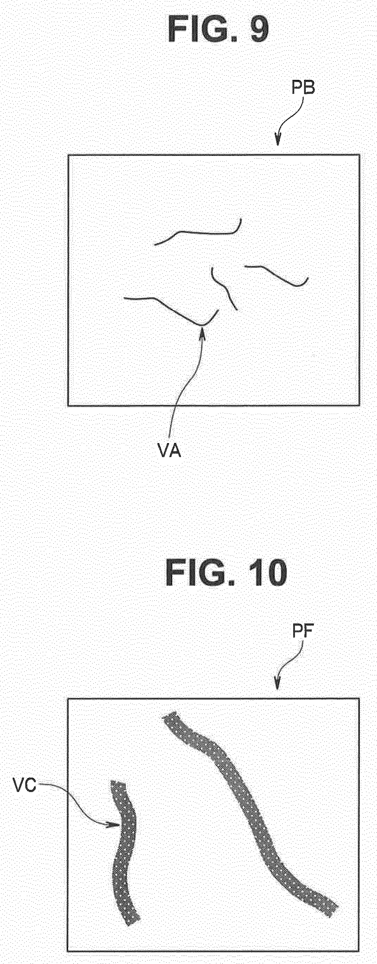

On the other hand, in the present embodiment, three display modes MDA, MDB, and MDC are provided as display modes for displaying the observation image generated by using at least one image of the intensity images PR, PG, and PB and a frequency spectrum image PF as illustrated in Fig. 10 to thereby improve the visibility of the blood vessel VC compared to the white light image IWL. A specific process and the like executed when one display mode of the display modes MDA, MDB, and MDC is set will be described below. Fig. 10 is a diagram schematically showing an example of the image generated by the image processing section of the biological observation system according to the embodiment. Note that specific description regarding the configurations and the like described above will be appropriately omitted for the simplification.

In the state that the distal end portion of the optical scanning probe 4 is arranged on the position that allows scanning the desirable biological tissue LM in the body cavity of the subject as a region to be observed, the user operates the display mode selector switch of the input device 11 to instruct the control section 12 to set one display mode of the display modes MDA, MDB, and MDC.

When the control section 12 senses that one display mode of the display modes MDA, MDB, and MDC is set, the control section 12 controls the light source section 2 to apply the R light, the G light, and the B light in time division and to illuminate the region to be observed in an illumination pattern IPL in which the application frequency of the R light is higher than both of the application frequency of the G light and the application frequency of the B light, for example. According to the control according to the illumination pattern IPL, the region to be observed is illuminated in the period of scanning for generating the observation image of one frame such that the number of times of application of the R light: the number of times of application of the G light: the number of times of application of the B light = 9 : 1 : 1, for example.

The control section 12 controls the image processing section 9 to switch the output destinations of the digital signals inputted to the selector 91 according to the illumination pattern IPL.

More specifically, the control section 12 controls the image processing section 9 to set the output destinations of the digital signals inputted to the selector 91 to both of the signal processing circuit 92 and the image generation circuit 93 according to the application timing of the R light in the illumination pattern IPL and set the output destination of the digital signals inputted to the selector 91 to the image generation circuit 93 according to the application timing of the G light and the B light in the illumination pattern IPL, for example.

According to the control by the control section 12 described above, the illumination light according to the illumination pattern IPL is applied along the scan path SP to two-dimensionally scan the region to be observed. The return light emitted from the region to be observed according to application of the illumination light enters the light detection section 8 through the optical fiber bundle 7. The digital signals generated according to the intensity of the return light are sequentially outputted to the image processing section 9.

According to the control by the control section 12 described above, the digital signals DRS generated by the light detection section 8 at the application timing of the R light in the illumination pattern IPL are outputted to the signal processing circuit 92 and the image generation circuit 93 through the selector 91. The digital signals DGS generated by the light detection section 8 at the application timing of the G light in the illumination pattern IPL are outputted to the image generation circuit 93 through the selector 91. The digital signals DBS generated by the light detection section 8 at the application timing of the B light in the illumination pattern IPL are outputted to the image generation circuit 93 through the selector 91.

The signal processing circuit 92 applies a process, such as DFT and FFT, to the digital signals DRS inputted through the selector 91 to generate frequency spectrum signals BFS indicating the frequency spectra obtained by analyzing the frequencies of the return light emitted from the region to be observed according to application of the R light and outputs the generated frequency spectrum signals BFS to the image generation circuit 93.

The image generation circuit 93 executes a mapping process or the like based on the control by the control section 12 to generate the intensity images PR, PG, and PB of one field and outputs the generated intensity images PR, PG, and PB to the display control circuit 94.

Based on the control by the control section 12, the image generation circuit 93 specifies the pixel positions in the raster format corresponding to the application positions of the R light in the scan path SP and generates the frequency spectrum image PF of one field by executing a mapping process or the like of mapping, on the specified pixel positions, the pixel information that is the luminance values according to the average values or the integrated values of the sizes of the frequency spectra indicated by the frequency spectrum signals BFS sequentially outputted from the signal processing circuit 92. The image generation circuit 93 outputs the generated frequency spectrum image PF to the display control circuit 94. That is, the frequency spectrum image PF is generated as an image with luminance values according to the sizes of scattering of the R light in which the frequency is shifted by scattering bodies, such as red blood cells, included in the blood flowing through the blood vessel VC.

When the control section 12 senses that the display mode MDA is set for example, the control section 12 controls the image processing section 9 to allocate the frequency spectrum image PF to the R channel of the display apparatus 10, allocate the intensity image PG to the G channel of the display apparatus 10, and allocate the intensity image PB to the B channel of the display apparatus 10.

When the display mode MDA is set, the display control circuit 94 generates an observation image of one frame by performing, for example, action of allocating the frequency spectrum image PF of one field outputted from the image generation circuit 93 to the R channel of the display apparatus 10, allocating the intensity image PG of one field outputted from the image generation circuit 93 to the G channel of the display apparatus 10, and allocating the intensity image PB of one field outputted from the image generation circuit 93 to the B channel of the display apparatus 10 and outputs the generated observation image to the display apparatus 10 based on the control by the control section 12.

According to the process and the like described above, the blood vessels VA and VB can be visually recognized when the display mode MDA is set, and the observation image in which the visibility of the blood vessel VC is improved compared to the white light image IWL is displayed on the display apparatus 10.

When the control section 12 senses that the display mode MDB is set for example, the control section 12 controls the image processing section 9 to allocate the image obtained by using the intensity image PR and the frequency spectrum image PF to the R channel of the display apparatus 10, allocate the intensity image PG to the G channel of the display apparatus 10, and allocate the intensity image PB to the B channel of the display apparatus 10.

When the display mode MDB is set, the display control circuit 94 generates an observation image of one frame by performing, for example, action of allocating, to the R channel of the display apparatus 10, the image of one field obtained as an arithmetic result of arithmetic processing using the intensity image PR of one field and the frequency spectrum image PF of one field outputted from the image generation circuit 93, allocating the intensity image PG of one field outputted from the image generation circuit 93 to the G channel of the display apparatus 10, and allocating the intensity image PB of one field outputted from the image generation circuit 93 to the B channel of the display apparatus 10 and outputs the generated observation image to the display apparatus 10 based on the control by the control section 12. Note that in the display mode MDB, the arithmetic processing executed to obtain the image allocated to the R channel of the display apparatus 10 may be, for example, an adding process of adding the intensity image PR and the frequency spectrum image PF, may be an averaging process of averaging the intensity image PR and the frequency spectrum image PF, or may be a dividing process of removing the intensity image PR from the frequency spectrum image PF.

According to the process and the like described above, the blood vessels VA and VB can be visually recognized when the display mode MDB is set, and the observation image in which the visibility of the blood vessel VC is further improved compared to the observation image of the display mode MDA is displayed on the display apparatus 10.

When the control section 12 senses that the display mode MDC is set for example, the control section 12 controls the image processing section 9 to allocate the intensity image PR to the R channel of the display apparatus 10, allocate the frequency spectrum image PF to the G channel of the display apparatus 10, and allocate the intensity image PB to the B channel of the display apparatus 10.

When the display mode MDC is set, the display control circuit 94 generates an observation image of one frame by performing, for example, action of allocating the intensity image PR of one field outputted from the image generation circuit 93 to the R channel of the display apparatus 10, allocating the frequency spectrum image PF of one field outputted from the image generation circuit 93 to the G channel of the display apparatus 10, and allocating the intensity image PB of one field outputted from the image generation circuit 93 to the B channel of the display apparatus 10 and outputs the generated observation image to the display apparatus 10 based on the control by the control section 12.

According to the process or the like described above, the blood vessels VA and VB can be visually recognized when the display mode MDC is set, and the observation image in which the visibility of the blood vessel VC is further improved compared to the observation image of the display mode MDB is displayed on the display apparatus 10.

Note that according to the present embodiment, when the display mode MDC is set, the control section 12 may perform control for illuminating the region to be observed in an illumination pattern IPT in which the R light and the B light are applied in time division, and the application frequencies of the R light are respectively higher than the application frequencies of the B light, for example.

According to the present embodiment, the control section 12 and the display control circuit 94 may perform the following action when the display mode MDC is set.

When the control section 12 senses that the display mode MDC is set, the control section 12 controls the image processing section 9 to allocate the intensity image PR to the R channel of the display apparatus 10, allocate the image obtained by using the intensity image PR and the frequency spectrum image PF to the G channel of the display apparatus 10, and allocate the intensity image PB to the B channel of the display apparatus 10, for example.

When the display mode MDC is set, the display control circuit 94 generates an observation image of one frame by performing, for example, action of allocating the intensity image PR of one field outputted from the image generation circuit 93 to the R channel of the display apparatus 10, allocating, to the G channel of the display apparatus 10, the image of one field obtained as an arithmetic result of arithmetic processing using the intensity image PR of one field and the frequency spectrum image PF of one field outputted from the image generation circuit 93, and allocating the intensity image PB of one field outputted from the image generation circuit 93 to the B channel of the display apparatus 10 and outputs the generated observation image to the display apparatus 10 based on the control by the control section 12. Note that in the display mode MDC, the arithmetic processing executed to obtain the image allocated to the G channel of the display apparatus 10 may be, for example, an adding process of adding the intensity image PR and the frequency spectrum image PF, may be an averaging process of averaging the intensity image PR and the frequency spectrum image PF, or may be a dividing process of removing the intensity image PR from the frequency spectrum image PF.

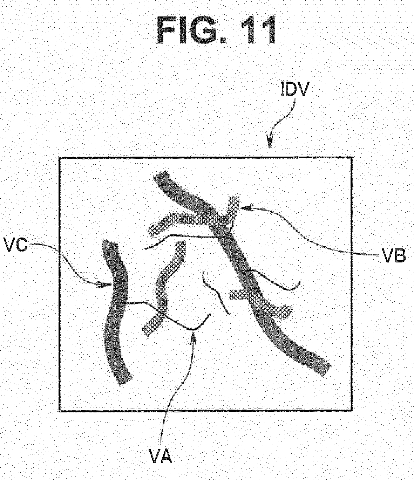

That is, according to the process and the like described above, when the display mode of one of the display modes MDA, MDB, and MDC is set, an observation image IDV in which the visibility of the blood vessel VC included in the desirable biological tissue LM is improved compared to the white light image IWL of Fig. 10 is displayed on the display apparatus 10 as shown for example in Fig. 11. Fig. 11 is a diagram schematically showing an example of the observation image displayed on the display apparatus of the biological observation system according to the embodiment.

As described, according to the present embodiment, the region to be observed including the desirable biological tissue LM is two-dimensionally scanned by the optical scanning probe 4, and the observation image that allows checking, all at once, a distribution state of the blood vessel VC existing in a deep part of the region to be observed is displayed on the display apparatus 10. Therefore, according to the present embodiment, when a surgical treatment is conducted for the desirable biological tissue LM for example, the treatment can be safely advanced while avoiding damage of the blood vessel VC. As a result, the burden of the surgeon conducting a surgical treatment of biological tissue in a body cavity can be reduced.

According to the present embodiment, the intensity image PR and the frequency spectrum image PF can be obtained all at once based on the return light emitted from the region to be observed according to application of the R light, and a frame rate in displaying the observation image on the display apparatus 10 can be substantially constant in each display mode. Therefore, according to the present embodiment, a visual unnatural feeling associated with switching of the display mode can be inhibited, and as a result, the burden of the surgeon conducting the surgical treatment of the biological tissue in the body cavity can be reduced.

According to the present embodiment, the region to be observed is illuminated in the illumination pattern IPL in which the application frequency of the R light is higher than both of the application frequency of the G light and the application frequency of the B light. Furthermore, the sizes of the frequency spectra included in the return light of the R light are integrated or averaged, and the frequency spectrum image PF with a high SN ratio can be generated. Therefore, according to the present embodiment, the observation image that allows easily recognizing the blood vessel VC can be displayed in the display modes MDA, MDB, and MDC, and as a result, the burden of the surgeon conducting the surgical treatment of the biological tissue in the body cavity can be reduced.

Note that the light detection device 81 of the present embodiment is not limited to the device (point detector), such as a PD and an APD, configured to generate electrical signals by receiving each point of the return light entering through the emission end portion of the optical fiber bundle 7. For example, the light detection device 81 may be a device (two-dimensional detector), such as a CCD and a CMOS, capable of generating electrical signals by two-dimensionally receiving the return light entering through the emission end portion of the optical fiber bundle 7.

The biological observation system 1 of the present embodiment is not limited to the system configured to two-dimensionally scan the region to be observed by applying the illumination light while swinging the emission end portion of the optical fiber 3. For example, the biological observation system 1 may be configured to two-dimensionally illuminate the region to be observed in a predetermined range all at once without swinging the emission end portion of the optical fiber 3.

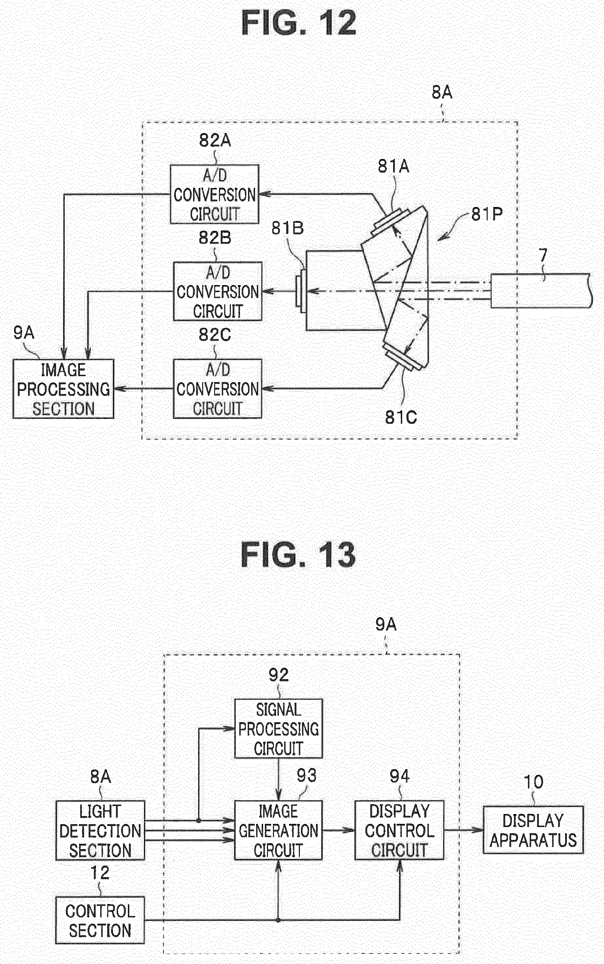

On the other hand, according to the present embodiment, the biological observation system 1 may be modified to include a light detection section 8A shown in Fig. 12 in place of the light detection section 8 and to include an image processing section 9A shown in Fig. 13 in place of the image processing section 9, for example. A configuration and the like regarding such a modification will be described below. Fig. 12 is a diagram for describing an example of a configuration of the light detection section of the biological observation system according to the modification of the embodiment. Fig. 13 is a diagram for describing an example of a configuration of the image processing section of the biological observation system according to the modification of the embodiment.

As shown in Fig. 12, the light detection section 8A includes a dichroic prism 81P, light detection devices 81A, 81B, and 81C, and A/D conversion circuits 82A, 82B, and 82C.

The dichroic prism 81P is, for example, a spectral device formed by combining a prism and a dichroic filter and is configured to separate the return light entering through the emission end portion of the optical fiber bundle 7 into respective wavelength bands of the R light, the G light, and the B light.

The light detection device 81A includes, for example, a PD or an APD and is configured to receive the R light included in the return light separated by the dichroic prism 81P, generate electrical signals according to the intensity of the received R light, and output the electrical signals to the A/D conversion circuit 82A.

The light detection device 81B includes, for example, a PD or an APD, and is configured to receive the G light included in the return light separated by the dichroic prism 81P, generate electrical signals according to the intensity of the received G light, and output the electrical signals to the A/D conversion circuit 82B.

The light detection device 81C includes, for example, a PD or an APD and is configured to receive the B light included in the return light separated by the dichroic prism 81P, generate electrical signals according to the intensity of the received B light, and output the electrical signals to the A/D conversion circuit 82C.

The A/D conversion circuit 82A is configured to convert the electrical signals outputted from the light detection device 81A to digital signals and sequentially output the digital signals to the image processing section 9A.

The A/D conversion circuit 82B is configured to convert the electrical signals outputted from the light detection device 81B to digital signals and sequentially output the digital signals to the image processing section 9A.

The A/D conversion circuit 82C is configured to convert the electrical signals outputted from the light detection device 81C to digital signals and sequentially output the digital signals to the image processing section 9A.

As shown in Fig. 13, the image processing section 9A has substantially the same configuration as the image processing section 9, except that the selector 91 is removed from the image processing section 9. Therefore, the digital signals outputted from the A/D conversion circuit 82A are inputted to both of the signal processing circuit 92 and the image generation circuit 93 in the image processing section 9A. The digital signals outputted from the A/D conversion circuit 82B and the digital signals outputted from the A/D conversion circuit 82C are both inputted to the image generation circuit 93 in the image processing section 9A.

The signal processing circuit 92 of the image processing section 9A is configured to apply a process, such as DFT and FFT, to the digital signals outputted from the A/D conversion circuit 82A to generate frequency spectrum signals indicating the frequency spectra obtained by analyzing the frequencies of the return light emitted from the region to be observed according to application of the illumination light and configured to output the generated frequency spectrum signals to the image generation circuit 93.

When the region to be observed is scanned in a predetermined scan path for example, the image generation circuit 93 of the image processing section 9A is configured to specify the pixel positions in a raster format corresponding to the application positions of the illumination light in the predetermined scan path based on the control by the control section 12. The image generation circuit 93 is configured to generate an image of one field by executing a mapping process or the like of mapping, on the specified pixel positions, pixel information that is luminance values according to the sizes of the gradation values of the digital signals sequentially outputted from the A/D conversion circuits 82A, 82B, and 82C and is configured to output the generated image to the display control circuit 94.

According to the configuration of the present modification described above, for example, even when the control section 12 performs control for illuminating the region to be observed in an illumination pattern IPM in which the R light and mixed light with a mixture of the G light and the B light are applied in time division, and the application frequency of the R light is higher than the application frequency of the mixed light, action for displaying the observation image corresponding to each of the display modes can be performed. Therefore, according to the configuration of the present modification described above, the observation image can be displayed at a frame rate higher than in the configuration of the embodiment described above in each of the display modes. Note that according to the control regarding the illumination pattern IPM, the region to be observed is illuminated in the period of performing scanning for generating the observation image of one frame such that the number of times of application of the R light: the number of times of application of the mixed light = 9 : 2, for example.

On the other hand, in the configuration according to the present modification described above, the control section 12 may perform control for illuminating the region to be observed not only in the illumination pattern IPM, but also in an illumination pattern IPN in which the R light, the G light, and the B light are applied at the same time, and the amount of applied light of the R light is higher than both of the amount of applied light of the G light and the amount of applied light of the B light, for example.

The present invention is not limited to the embodiment and the modification, and it is obvious that various changes and applications are possible without departing from the scope of the invention.

The present application is filed on the basis of claiming the benefit of priority from Japanese Patent Application No. 2015-86978, filed in Japan on April 21, 2015, and the disclosed content is incorporated in the present specification, claims, and drawings by reference.

## Claims

1. A biological observation system comprising:
a light source section configured to supply illumination light applied to a region to be observed including biological tissue, the illumination light including at least first illumination light that is light of one of red light and near infrared light in a visible region and second illumination light that is light in the visible region different from the first illumination light;
a light detection section configured to receive return light emitted from the region to be observed according to application of the illumination light and output an output signal according to intensity of the received return light;
a signal processing section configured to analyze a frequency of the output signal outputted from the light detection section to extract a frequency spectrum signal indicating a frequency spectrum of the return light emitted from the region to be observed according to application of the first illumination light;
an image generation section configured to generate one or more intensity images based on the output signal outputted from the light detection section according to the application of the illumination light and generate a frequency spectrum image based on the frequency spectrum signal outputted from the signal processing section according to the application of the first illumination light; and
a display control section configured to perform action for generating and displaying an observation image of the region to be observed by using at least one intensity image of the one or more intensity images and the frequency spectrum image.

2. The biological observation system according to claim 1, wherein
the light source section is configured to supply the illumination light including the first illumination light, the second illumination light, and third illumination light that is light in the visible region different from both of the first illumination light and the second illumination light.

3. The biological observation system according to claim 2, further comprising
a control section configured to control the light source section to apply the first illumination light, the second illumination light, and the third illumination light to the region to be observed in time division and set an application frequency of the first illumination light higher than both of an application frequency of the second illumination light and an application frequency of the third illumination light, wherein
the image generation section is configured to generate three intensity images based on the output signal outputted from the light detection section according to the application of the first illumination light, the second illumination light, and the third illumination light, and
the display control section is configured to perform action for generating and displaying an observation image of the region to be observed by using at least one intensity image of the three intensity images and the frequency spectrum image.

4. The biological observation system according to claim 2, further comprising
a control section configured to perform control for applying the first illumination light and mixed light with a mixture of the second illumination light and the third illumination light to the region to be observed in time division and setting an application frequency of the first illumination light higher than an application frequency of the mixed light, wherein
the image generation section is configured to generate three intensity images based on the output signal outputted from the light detection section according to the application of the first illumination light and the mixed light, and
the display control section is configured to perform action for generating and displaying an observation image of the region to be observed by using at least one intensity image of the three intensity images and the frequency spectrum image.

5. The biological observation system according to claim 2, further comprising
a control section configured to perform control for applying the first illumination light, the second illumination light, and the third illumination light to the region to be observed at a same time and setting an amount of applied light of the first illumination light higher than both of an amount of applied light of the second illumination light and an amount of applied light of the third illumination light, wherein
the image generation section is configured to generate three intensity images based on the output signal outputted from the light detection section according to the application of the illumination light, and
the display control section is configured to generate and display an observation image of the region to be observed by using at least one intensity image of the three intensity images and the frequency spectrum image.

6. The biological observation system according to claim 1, further comprising
an optical scanning probe configured to scan the region to be observed by displacing an application position of the illumination light supplied from the light source section along a predetermined scan path and transmit return light emitted from the region to be observed to the light detection section.
